(19) Europäisches Patentamt
European Patent Office
Office européen des brevets

(11) Numéro de publication : **0 047 207**
**B1**

(12) **FASCICULE DE BREVET EUROPÉEN**

(45) Date de publication du fascicule du brevet : **20.02.85**

(51) Int. Cl.⁴ : **C 07 D207/09**, A 61 K 31/40

(21) Numéro de dépôt : **81401340.5**

(22) Date de dépôt : **26.08.81**

(54) **N-(1-allyl 2-pyrrolidinyl méthyl) 2-méthoxy 4-amino 5-méthylsulfamoyl benzamide, son procédé de préparation et son application comme médicament.**

(30) Priorité : **28.08.80 FR 8018635**

(43) Date de publication de la demande : **10.03.82 Bulletin 82/10**

(45) Mention de la délivrance du brevet : **20.02.85 Bulletin 85/08**

(84) Etats contractants désignés : **AT BE CH DE FR GB IT LI LU NL SE**

(56) Documents cités :
**FR-M- 5 916**
**L.F. FIESER et al. "Reagents for organic synthesis" JOHN WILEY AND SONS INC. 1967 NEW YORK (US)**

(73) Titulaire : **SOCIETE D'ETUDES SCIENTIFIQUES ET INDUSTRIELLES DE L'ILE DE FRANCE**
**46, Boulevard de Latour-Maubourg**
**F-75340 Paris Cedex 07 (FR)**

(72) Inventeur : **Perrot, Jacques**
**15, avenue Emile Deschanel**
**F-75007 Paris (FR)**
Inventeur : **Thominet, Michel**
**82, rue Bonaparte**
**F-75006 Paris (FR)**

(74) Mandataire : **Gallochat, Alain**
**SOCIETE D'ETUDES SCIENTIFIQUES ET INDUSTRIELLES DE L'ILE DE FRANCE 46, Boulevard de Latour Maubourg**
**F-75340 Paris Cedex 07 (FR)**

**EP 0 047 207 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un nouveau benzamide, à savoir le N-(1-allyl 2-pyrrolidinyl méthyl) 2-méthoxy 4-amino 5-méthylsulfamoyl benzamide, ainsi qu'un procédé de préparation et son application comme médicament, en particulier pour le traitement de fond de la migraine. Dans la suite de la description, le N-(1-allyl 2-pyrrolidinyl méthyl) 2-méthoxy 4-amino 5-méthylsulfamoyl benzamide sera dénommé benzamide selon l'invention, ledit benzamide répondant à la formule suivante :

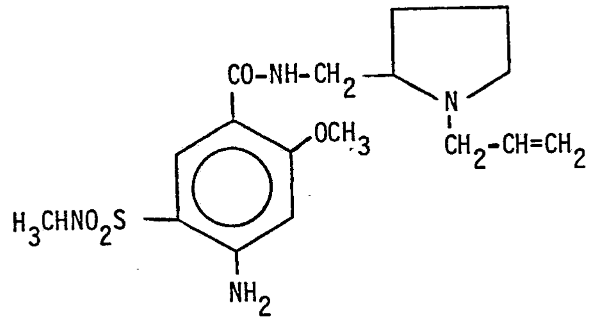

La présente invention couvre ledit benzamide selon l'invention, les sels d'ammonium quaternaires du benzamide selon l'invention, ses N-oxydes, ses isomères optiques ainsi que leurs sels d'addition d'acides pharmacologiquement acceptables.

D'après le brevet Fr. 5916 M, on connaît des benzamides hétérocycliques substitués, en particulier des dérivés de N-(2-pyrrolidinylméthyl)-benzamides substitués sur l'atome d'azote de la pyrrolidine et sur le noyau benzénique.

Ces benzamides sont actifs comme agents antiémétiques et tranquillisants.

Le composé de la présente invention, dont la structure est voisine de celle des composés décrits dans le brevet 5916 M, mais qui correspond à la combinaison nouvelle de la 1-allyl 2-aminométhyl pyrrolidine et de l'acide 2-méthoxy 4-amino 5-méthylsulfamoyl benzoïque, s'est révélé actif comme agent antimigraineux, activité tout à fait inattendue d'après les propriétés connues des composés du brevet 5916 M.

Un procédé d'obtention du benzamide selon l'invention va maintenant être décrit, la matière de départ étant le 2-méthoxy 4-acétamino benzoate de méthyl connu par ailleurs :

1. 2-Méthoxy 4-acétamino 5-sulfo benzoate de méthyle

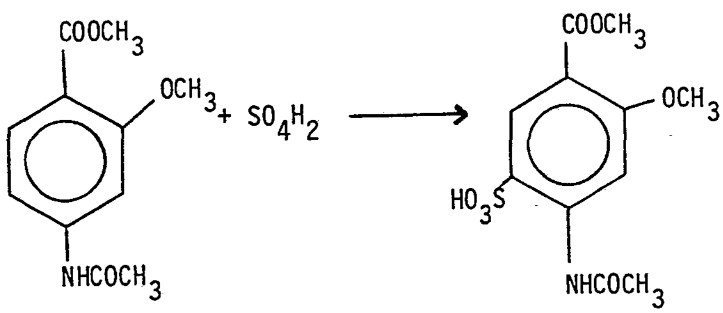

Dans un réacteur de 100 litres on charge 45 litres d'acide acétique. On agite et ajoute 22,5 kg de 2-méthoxy 4-acétamino benzoate de méthyle puis on charge 30 litres d'anhydride acétique.

On introduit, en 15 minutes, 5,730 litres d'acide sulfurique (d : 1,84). La température monte d'elle-même à 50 °C. L'ester se dissout.

On porte lentement la température à 70 °C. L'acide sulfonique cristallise ; lorsque l'on a atteint 70 °C, on refroidit aussitôt, à 20 °C, et on essore sur essoreuse.

Le produit est lavé avec deux fois 8 litres d'acétone, puis séché en étuve à 50 °C.

Caractéristiques du produit obtenu :

— Poids : 24,5 kg
— Rendement : 81,5 %
— Fusion : 240 °C avec décomposition
— S % : (théorie : 10,57 %) 10,56 %
— Cristaux blancs

2. 2-Méthoxy 4-amino 5-sulfo benzoate de méthyle

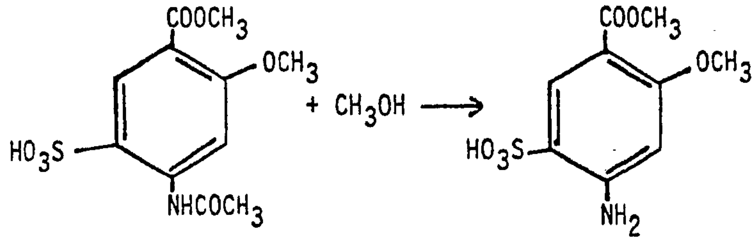


Dans un réacteur de 100 litres on charge 75 litres de méthanol. On agite et ajoute 24,5 kg de 2-méthoxy 4-acétamino 5-sulfo benzoate de méthyle finement broyé.

L'acide sulfonique se dissout ; peu après, le dérivé désacétylé cristallise. Le mélange réactionnel est abandonné 24 heures à température ambiante, avec agitation, de manière à terminer la précipitation.

Le produit est essoré sur essoreuse, lavé avec 2 × 6 litres d'alcool méthylique et séché en étuve ventilée à 50 °C ; les caractéristiques de ce produit sont les suivantes :

— Poids : 20,5 kg
— Rendement : 98 %
— Fusion : 240 °C avec décomposition
— S % : 12,10 %
— Théorie : 12,26 %

3. 2-Méthoxy 4-amino 5-méthylsulfamoyl benzoate de méthyle

a)

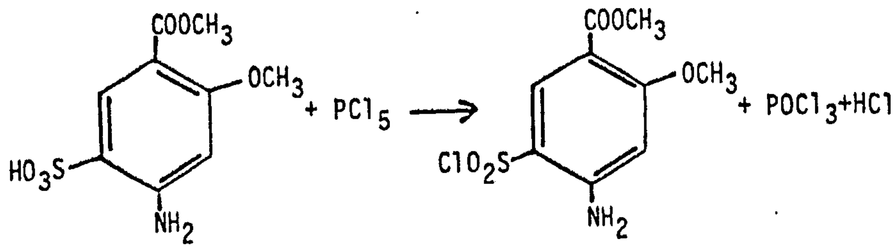


b)

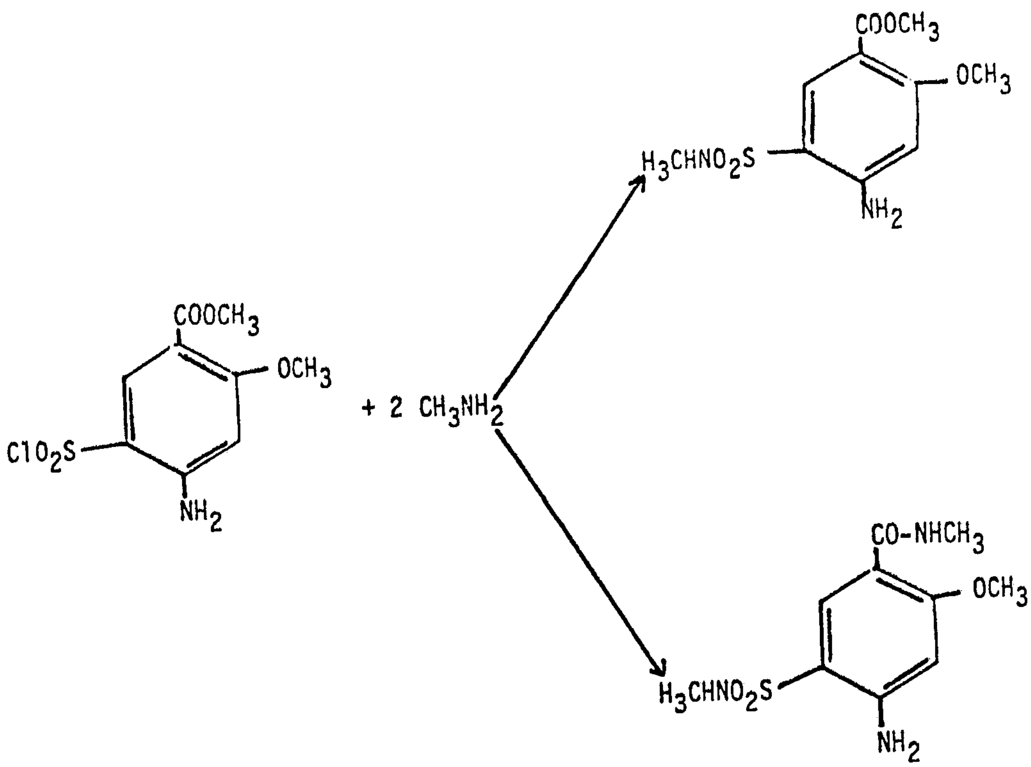

a) Dans un réacteur de 100 litres on charge 33,5 litres d'acétonitrile.

On agite, et on ajoute 16,8 kg de 2-méthoxy 4-amino 5-sulfo benzoate de méthyle ; par fractions, en 15 minutes environ, 17,5 kg de pentachlorure de phosphore sont également ajoutés.

On chauffe progressivement vers 80-85 °C température du reflux. La réaction démarre vers 30 °C ; on note alors un fort dégagement gazeux. En deux heures on atteint 80 °C.

Le reflux est maintenu pendant 1 heure 30. On refroidit cette solution à 55 °C.

b) Dans un réacteur de 200 litres on introduit 95 litres d'une solution aqueuse à 35 % de méthylamine. On agite et on refroidit à − 15 °C. On coule alors la solution précédente (sulfochlorure) lentement et par fractions, en maintenant une température inférieure à 10-15 °C.

L'introduction dure 4 heures puis on laisse la température remonter à 20 °C. La solution est diluée avec 300 litres d'eau, puis filtrée avec 2 kg de noir végétal.

Le filtrat est acidifié, sous agitation avec 70 litres d'acide chlorhydrique (d = 1,18).

Le produit cristallise peu à peu. On l'abandonne 120 heures au repos. Il est essoré, lavé à l'eau et séché en étuve à 50 °C.

On obtient ainsi 15 kg de ce produit.

4. Acide 2-méthoxy 4-amino 5-méthyl sulfamoyl benzoïque

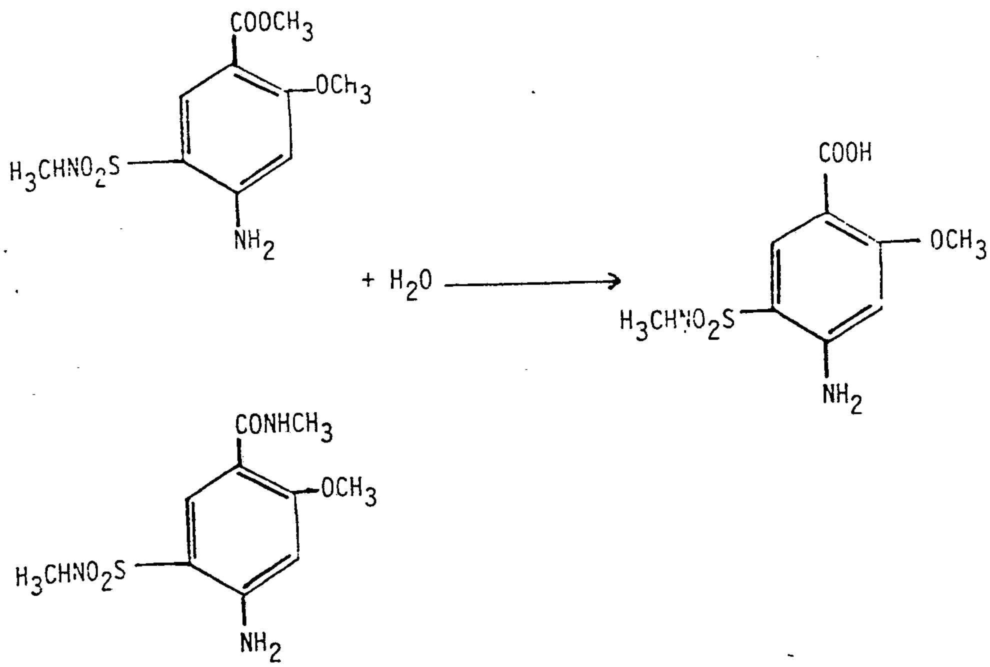


Dans un réacteur de 100 litres on charge 22 litres d'eau et 22 litres de lessive de soude, et sous agitation 15 kg du produit précédent.

On porte au reflux-franc pendant 20 heures.

On note un fort dégagement de méthylamine pendant les premières heures de réaction.

On refroidit à 20 °C et on verse la solution dans la cuve à précipitation.

On ajoute 12 litres d'eau. On agite et on acidifie à pH 1 avec 17 litres d'acide chlorhydrique d = 1,18. Il est nécessaire de refroidir pendant la coulée de l'acide.

L'acide cristallise ; il est essoré à 20 °C, lavé à l'eau et séché en étuve à 60 °C. Ses caractéristiques sont les suivantes :

— Poids : 12,100 kg
— PF : 205 °C
— IA : théorie 215,4 — obtenu : 212
— S % : théorie 12,31 % — obtenu : 12,23 %

Dans la cuve à précipitation on introduit 40 litres d'eau et 2,600 kg de bicarbonate de sodium. On agite et on ajoute par portions 12,100 kg d'acide 2-méthoxy 4-amino 5-méthyl sulfamoyl benzoïque. L'acide se dissout avec formation de mousses importantes.

Il persiste un léger insoluble gélatineux, éliminé par filtration sous vide.

Le filtrat est acidifié par 5,6 litres d'acide chlorhydrique.

Le produit cristallise ; il est essoré, lavé à l'eau et séché en étuve à 60 °C. Les caractéristiques de l'acide purifié sont alors :

— Poids : 11,9 kg
— Fusion : 202 °C
— IA : théorie 215,4 — obtenu : 215
— S % : théorie 12,31 % — obtenu : 12,37 %

5. N-(1-allyl 2-pyrrolidinyl méthyl) 2-méthoxy 4-amino 5-méthyl sulfamoyl benzamide

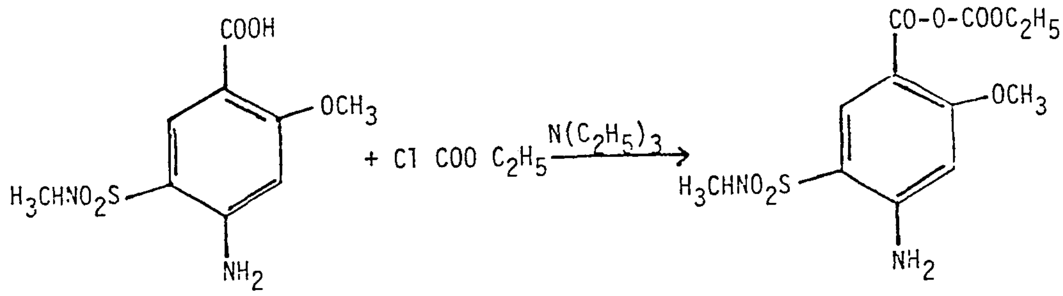


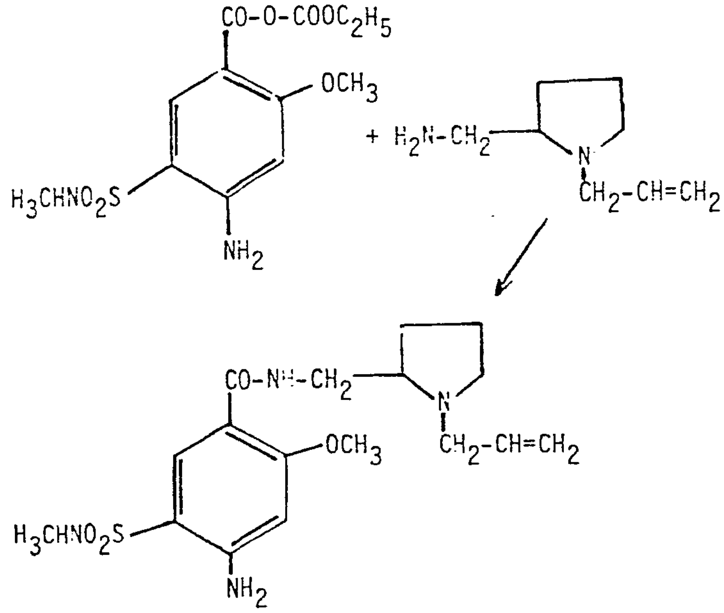

Dans un réacteur de 100 litres, on charge 10 litres d'eau permutée. On agite, puis on charge 5 300 cm$^3$ de triéthylamine et 10 kg d'acide 2-méthoxy 4-amino 5-méthyl sulfamoyl benzoïque.

On chauffe la suspension vers 45 °C pour tout dissoudre, puis on refroidit à + 5 °C par un courant de saumure et on ajoute 25 litres d'acétone.

Sans dépasser + 10 °C on verse lentement 3 670 cm$^3$ de chloroformiate d'éthyle (durée de l'introduction 15 minutes). On maintient l'agitation 30 minutes à cette température. Sans dépasser + 15 °C, on verse lentement 6,4 kg de N-allyl 2-aminométhyl pyrrolidine (durée de l'introduction 30 minutes).

On laisse la température remonter à 20 °C et on agite 1 heure à cette température. L'acétone est distillée à pression ordinaire puis sous vide, sans dépasser 60 °C dans la masse.

On ajoute 55 litres d'eau et on acidifie à pH 3-4 par 7 litres d'acide chlorhydrique chimiquement pur, et on ajoute 1 kg de noir actif carbone 3S. La solution est filtrée. Le filtrat est alcalinisé, sous forte agitation par 5 litres d'ammoniaque. On ajoute 10 kg de glace et on maintient l'agitation pendant 1 heure. Le benzamide cristallise d'abord liquide. On laisse au repos une nuit.

Il est essoré à 20 °C, lavé à l'eau et séché à 60 °C en étuve, avec les caractéristiques suivantes :

— Poids : 9 kg
— Rendement : 61 %
— Fusion : 166-167 °C

Dans un réacteur de 50 litres on dissout à ébullition 9 kg du benzamide précédemment obtenu dans 18 litres d'alcool éthylique absolu. La solution est filtrée avec 1 kg de noir végétal, sur filtre à pression.

Le filtrat est lavé avec 2 litres d'alcool bouillant, puis est refroidi à 10 °C. Le benzamide cristallise. Il est essoré, lavé à l'alcool froid et séché en étuve à 60 °C, avec les caractéristiques suivantes :

— Cristaux blancs
— Poids : 8 kg
— Rendement de recristallisation : 89 %

L'étude chromatographique révélant des taches importantes il sera nécessaire de procéder à deux nouvelles recristallisations dans l'alcool éthylique absolu, aboutissant ainsi à un produit purifié de caractéristiques suivantes :

— Cristaux blancs
— Poids : 6 kg
— Rendement des recristallisations : 67 %

Le procédé tel qu'il vient d'être décrit aboutit au benzamide selon l'invention avec un rendement global de 41 %. L'analyse du benzamide ainsi obtenu a donné les résultats suivants :

— Point de fusion : 168,5-169 °C
— Titre : 99,8 %
— $H_2O$ % : 0,1
— C % : théorie 53,38 — obtenu : 53,13. — H % : théorie 6,85 — obtenu : 6,88
— N % : théorie 14,65 — obtenu : 14,65. — S % : théorie 8,58 — obtenu : 8,54

Les spectres dans leur ensemble, sont compatibles avec la structure proposée.
L'étude chromatographique révèle l'existence d'une tache secondaire estimée entre 0,2 et 0,5 %.
Le benzamide selon l'invention, a fait l'objet d'une étude pharmacologique. La toxicité aiguë du composé a d'abord été déterminée et les valeurs des DL 50 par différentes voies d'administration sont indiquées ci-après :

| Produit | Voie | DL 50 souris mâle mg/kg (base) |
|---|---|---|
| Benzamide selon l'invention | I.V. | 44 |
| | I.P. | 184 |
| | S.C. | 204 |
| | P.O. | 3600 |

Les effets du benzamide sur le système nerveux central et notamment une action de type neuroleptique ont été recherchés.
Les essais ont montré que le composé était très peu dépresseur ainsi :
il ne réduit que très faiblement la motilité spontanée de la souris, même à dose élevée (Tableau 1)
il ne prolonge pas la durée de l'hypnose barbiturique (Tableau 2)

Tableau 1

| Produit | Voie | Motilité spontanée de la souris : effet inhibiteur observé à la dose maximale administrée | |
|---|---|---|---|
| | | Technique de WINTER et FLATAKER | Technique de l'activographe |
| Benzamide selon l'invention | I.P. | Effet de 26 % à 80 mg/kg | Effet de 22 % à 80 mg/kg |
| | P.O. | Effet de 20 % à 300 mg/kg | Effet de 25 % à 300 mg/kg |

Tableau 2

| Produit | Voie | Dose mg/kg | Temps d'hypnose souris traitées / Temps d'hypnose souris témoins |
|---|---|---|---|
| Benzamide selon l'invention | I.P. | 80 | 1,6 |

N. B. Le barbiturique utilisé est le pentobarbital à la dose de 60 mg/kg par voie intrapéritonéale.

A la différence des neuroleptiques classiques, le composé ne provoque pas de catalepsie chez le rat, même à la dose de 200 mg/kg s.c. et n'antagonise pas les mouvements stéréotypiques provoqués chez le rat par des agonistes dopaminergiques, tels que l'apomorphine ou l'amphétamine.

D'autre part, le benzamide est dépourvu d'action anticonvulsivante quand les convulsions sont provoquées par un choc électrique, un agent chimique (pentétrazol, nicotine) ou par un stimulus auditif.

L'effet analgésique du benzamide, en administration unique, est faible à l'égard de la douleur provoquée chez la souris par un stimulus mécanique, chimique ou thermique.

Les effets du benzamide sur le système cardiovasculaire ont été étudiés chez le chien anesthésié au chloralose, avec les résultats suivants :

— Le composé provoque une baisse de la pression artérielle proportionnelle à la dose administrée (Tableau 3).

— Le composé ne modifie pas la réponse hypotensive à l'acétylcholine ou à l'excitation du vague. L'absence d'effet anticholinergique du benzamide a été confirmée par un essai in vitro sur l'iléon isolé du cobaye.

— Le composé réduit les réponses hypertensives provoquées par les catécholamines (adrénaline et noradrénaline), par l'occlusion des carotides et par de faibles doses de nicotine (Tableau 4).

— Le composé diminue la réponse hypertensive à la sérotonine chez le chien anesthésié et traité en outre par un ganglioplégique, la chlorisondamine, qui stabilise cette réponse (Tableau 5).

Tableau 3

| Dose cumulative (mg/kg/I.V.) du benzamide selon l'invention | Variations de la pression artérielle par rapport à la pression initiale |
|---|---|
| 0,5 | - 21 % |
| 1 | - 32 % |
| 2 | - 36 % |
| 4 | - 48 % |
| 8 | - 54 % |
| 16 | - 59 % |
| 32 | - 58 % |

(Voir Tableaux page suivante)

Tableau 4

| | Techniques | Dose cumulative (mg/kg/I.V.) du benzamide selon l'invention | |
|---|---|---|---|
| Système orthosympathique | Occlusion des carotides (pendant 30 secondes)<br><br>% de réduction par rapport à l'hypertension témoin (R) | 0,5<br>1<br>2<br>4<br>8<br>16<br>32 | 0 %<br>R = 30 %<br>R = 43 %<br>R = 48 %<br>R = 86 %<br>R = 90 %<br>R =100 % |
| | Adrénaline<br><br>% de réduction par rapport à l'hypertension témoin (R) | 0,5<br>1<br>2<br>4<br>8<br>16<br>32 | R = 5 %<br>R = 3 %<br>R = 4 %<br>R = 21 %<br>R = 40 %<br>R = 68 %<br>R = 84 % |
| | Noradrénaline<br><br>% de réduction par rapport à l'hypertension témoin (R) | 0,5<br>1<br>2<br>4<br>8<br>16<br>32 | R = 21 %<br>R = 13 %<br>R = 30 %<br>R = 30 %<br>R = 40 %<br>R = 49 %<br>R = 66 % |
| | Nicotine<br><br>% de réduction de la composante orthosympatique (R) | 0,5<br>1<br>2<br>4<br>8<br>16<br>32 | 0 %<br>0 %<br>0 %<br>0 %<br>0 %<br>R = 49 %<br>R = 79 % |

Tableau 5

| Produit | Doses en mg/kg I.V. | Sérotonine en µg/kg I.V. | % inhibition de l'hypertension après | | | | | |
|---|---|---|---|---|---|---|---|---|
| | | | 3 mn | 30 mn | 1 h | 1 h 30 | 2 h | 3 h |
| Benzamide selon l'invention LOT 1 | 5 | 25 | 62 % | 63 % | 51 % | 51 % | 40 % | - |
| | 1 | 37,5 | 22 % | 8 % | - | - | - | - |
| Benzamide selon l'invention LOT 2 | 5 | 25 | 78 % | 65 % | 48 % | 43 % | 33 % | 33 % |
| | 1 | 25 | 36 % | 11 % | 0 | - | - | - |

Les résultats résumés dans le Tableau 5 ci-dessus ayant montré un antagonisme exercé par le benzamide selon l'invention sur l'effet presseur de la sérotonine, d'autres interactions possibles avec la sérotonine ont été recherchées.

Les essais ont montré que le benzamide selon l'invention :

— inhibait la contraction de l'utérus isolé de ratte, provoquée par la sérotonine. La dose inhibitrice 50 de la contraction est de l'ordre de 0,2 mg/l.

— exerçait un effet protecteur à l'égard de l'action ulcérogène gastrique de la sérotonine chez le rat, la dose efficace 50 étant de l'ordre de 3 mg/kg s.c.

— diminuait l'œdème de la patte du rat qui avait reçu une injection intraplantaire de 0,01 mg de sérotonine. Par voie i.p., la DE 50 est de l'ordre de 4 à 6 mg/kg et de 200 mg par voie orale.

Enfin, une interaction du benzamide selon l'invention avec l'histamine a été recherchée sur la pression artérielle du chien anesthésié et sur l'iléon isolé de cobaye. Aucune propriété antihistaminique n'a été mise en évidence par les deux tests.

Etant donné le profil pharmacologique du benzamide selon l'invention, défini par les tests ci-dessus rapportés, il est apparu que la composante d'action antisérotonine d'une part, l'effet circulatoire d'autre part, pouvaient conférer à ce benzamide un intérêt thérapeutique dans les migraines où précisément semblent intervenir des troubles vasomoteurs relevant des transmissions adrénergiques et sérotoninergiques, ce dernier médiateur pouvant agir aussi par d'autres mécanismes.

Une recherche clinique a montré le bien fondé de cette hypothèse.

La première étude de pharmacologie clinique consistait à explorer par une méthode non invasive l'hémodynamique cérébrale chez les migraineux, par enregistrement de la pulsatilité des artères cérébrales. Les tracés ont montré des anomalies permanentes par rapport à un témoin normal en dehors des crises, permettant une définition objective du malade migraineux. Des tracés ont ainsi été réalisés après absorption du benzamide selon l'invention par des migraineux.

Sous traitement par ledit benzamide à dose unique de 100 mg injectée par voie intramusculaire, le tracé évolue en 2 h vers la normalisation. Celle-ci se maintient sous traitement à doses répétées (150 mg/jour) par voie orale pendant 3 semaines.

L'intervention du benzamide selon l'invention dans la correction des anomalies de l'hémodynamique cérébrale chez les migraineux, ayant été montrée, les études cliniques ont été développées comprenant :

1) Une étude comparative en double aveugle croisé, le produit de référence étant l'oxétorone, médicament connu, appliqué dans la migraine. L'étude a porté sur 63 malades migraineux, les deux traitements étant appliqués pendant 30 jours avec une interruption suffisante entre les séquences.

La dose de benzamide a été de 150 mg par jour, celle de l'oxétorone de 120 mg, selon les recommandations de la littérature.

L'analyse des résultats, tenant compte à la fois de l'efficacité thérapeutique et de la tolérance du traitement, indique que le benzamide selon l'invention est statistiquement supérieur à l'oxétorone ($P < 0.05$).

2) Une étude comparative avec le Pizotifène, composé antihistaminique et antisérotonine, également appliqué dans le traitement de fond de la migraine.

Cette étude a porté sur 17 malades présentant des crises de migraines vraies de fréquence régulière permettant d'apprécier l'efficacité d'un traitement d'après le nombre et l'intensité des crises au cours d'une période d'un mois.

Le benzamide selon l'invention a été appliqué par voie orale à la dose de 150 mg par jour, en 3 prises.

Le Pizotifène a également été administré par voie orale à la posologie progressive préconisée pour ce produit (0,73 mg × 1 pendant 3 jours — 0,73 mg × 2 pendant 3 jours — 0,73 mg × 3 pendant 24 jours).

La répartition des malades selon les traitements a été effectuée par randomisation.

L'analyse des résultats confirme l'activité du benzamide selon l'invention, qui s'est révélé plus efficace que ce deuxième composé de référence (P # 0.02).

3) Une étude ouverte portant sur 80 malades, suivis en traitement ambulatoire à raison de 150 mg du benzamide par jour par voie bucale en 3 prises :

79 % de succès ont été enregistrés et dans des cas particulièrement sévères de migraines « accompagnées » la rémission a transformé la vie sociale des malades. La tolérance générale du traitement a été très satisfaisante.

En conclusion, le benzamide selon l'invention présente un intérêt thérapeutique démontré dans le traitement de fond de la migraine.

**Revendications** (pour les Etats contractants : BE, CH, DE, FR, GB, IT, LI, LU, NL, SE)

1. A titre de produit nouveau, le N-(1-allyl 2-pyrrolidinyl méthyl) 2-méthoxy 4-amino 5-méthylsulfamoyl benzamide, ses sels d'ammonium quaternaires, ses N-oxydes, ses isomères optiques ainsi que leurs sels d'addition d'acides pharmacologiquement acceptables.

2. Procédé de synthèse du produit selon la revendication 1, caractérisé en ce qu'il consiste à faire réagir le chloroformiate d'éthyle sur l'acide 2-méthoxy 4-amino 5-méthylsulfamoyl benzoïque en présence de triéthylamine, puis à faire réagir la N-allyl 2-aminométhyl pyrrolidine sur l'anhydride mixte ainsi obtenu.

3. Procédé selon la revendication 2 caractérisé en ce que l'acide 2-méthoxy 4-amino 5-méthylsulfamoyl benzoïque est fabriqué à partir du 2-méthoxy 4-acétamino benzoate de méthyl par action successive d'acide sulfurique, de méthanol, de $PCl_5$, de méthylamine, puis en hydrolysant le produit ainsi obtenu.

4. A titre de médicament nouveau, le benzamide selon la revendication 1.
5. Médicament selon la revendication 4 pour le traitement de fond de la migraine.
6. Composition pharmaceutique caractérisée en ce qu'elle contient comme principe actif le benzamide de la revendication 1 en association avec un support pharmaceutiquement acceptable.

**Revendications** (pour l'État contractant AT)

1. Procédé de synthèse du N-(1-allyl 2-pyrrolidinyl méthyl) 2-méthoxy 4-amino 5-méthylsulfamoyl benzamide, ses sels d'ammonium quaternaires, ses N-oxydes, ses isomères optiques ainsi que leurs sels d'addition d'acides pharmacologiquement acceptables, qui consiste à faire réagir le chloroformiate d'éthyle sur l'acide 2-méthoxy 4-amino 5-méthylsulfamoyl benzoïque en présence de triéthylamine, puis à faire réagir la N-allyl 2-aminométhyl pyrrolidine sur l'anhydride mixte ainsi obtenu.
2. Procédé selon la revendication 1, caractérisé en ce que le N-(1-allyl 2-pyrrolidinyl méthyl)-2-méthoxy 4-amino 5-méthylsulfamoyl benzamide est transformé en sel d'addition au moyen d'un acide organique ou minéral, en sel d'ammonium quaternaire au moyen d'un sel d'alkyle, ou oxydé en N-oxyde.
3. Procédé selon la revendication 1 caractérisé en ce que l'on obtient le benzamide sous forme optiquement active, par traitement avec un acide optiquement actif.
4. Procédé selon la revendication 1 caractérisé en ce que l'acide 2-méthoxy 4-amino 5-méthylsulfamoyl benzoïque est fabriqué à partir du 2-méthoxy 4-acétamino 5-sulfobenzoate de méthyle par action successive d'acide sulfurique, de méthanol, de $PCl_5$, de méthylamine, puis en hydrolysant le produit ainsi obtenu.

**Claims** (for the Contracting States : BE, CH, DE, FR, GB, IT, LI, LU , NL, SE)

1. As a novel product, N-(1-allyl-2-pyrrolidinyl methyl)-2-methoxy-4-amino-5-methylsulfamoyl benzamide, its quaternary ammonium salts, its N-oxides, its optical isomers and their pharmacologically acceptable acid addition salts.
2. A process for synthesis of the product according to claim 1, characterised in that it comprises reacting ethyl chloroformate on 2-methoxy-4-amino-5-methylsulfamoyl benzoic acid in the presence of triethylamine, then reacting N-allyl-2-aminomethyl pyrrolidine on the mixed anhydride produced in that way.
3. A process according to claim 2, characterised in that 2-methoxy-4-amino-5-methylsulfamoyl benzoic acid is produced from methyl 2-methoxy-4-acetamino benzoate by the successive action of sulfuric acid, methanol, $PCl_5$ and methylamine and hydrolysing the resulting product.
4. As a novel medicament, the benzamide according to claim 1.
5. A medicament according to claim 4 applied to the basic treatment of migraine.
6. A pharmaceutical composition characterised in that it contains as its active ingredient the benzamide of claim 1 in association with a pharmaceutically acceptable carrier.

**Claims** (for the Contracting State AT)

1. A process for synthesis of N-(1-allyl-2-pyrrolidinylmethyl)-2-methoxy-4-amino-5-methylsulfamoyl benzamide, its quaternary ammonium salts, its N-oxides, its optical isomers and their pharmacologically acceptable acid addition salts, which comprises reacting ethyl chloroformate on 2-methoxy-4-amino-5-methylsulfamoyl benzoic acid in the presence of triethylamine, then reacting N-allyl-2-aminomethyl pyrrolidine on the mixed anhydride which is produced in that way.
2. A process according to claim 1, characterised in that the N-(1-allyl-2-pyrrolidinylmethyl)-2-methoxy-4-amino-5-methylsulfamoyl benzamide is converted into an addition salt by means of an organic or inorganic acid, into a quaternary ammonium salt by means of an alkyl salt, or oxidised to N-oxide.
3. A process according to claim 1, characterised in that the benzamide is produced in optically active form by treatment with an optically active acid.
4. A process according to claim 1, characterised in that the 2-methoxy-4-amino-5-methylsulfamoyl benzoic acid is produced from methyl 2-methoxy-4-acetamino-benzoate by the successive action of sulfuric acid, methanol, $PCl_5$ and methylamine, and then hydrolysing the resulting product.

**Ansprüche** (für die Vertragsstaaten : BE, CH, DE, FR, GB, IT, LI, LU, NL, SE)

1. N-(1-Allyl-2-pyrrolidinylmethyl)-2-methoxy-4-amino-5-methylsulfamoylbenzamid als neue Verbindung, und quartäre Ammoniumsalze, N-Oxide, optische Isomere sowie pharmakologisch verträgliche Säureadditionssalze desselben.
2. Verfahren zur Herstellung der Verbindung nach Anspruch 1, dadurch gekennzeichnet, daß man

Chlorameisensäure-ethylester mit 2-Methoxy-4-amino-5-methylsulfamoylbenzoesäure in Gegenwart von Triethylamin reagieren läßt und anschließend N-Allyl-2-aminomethylpyrrolidin mit dem so erhaltenen gemischten Anhydrid umsetzt.

3. Verfahren nach Anspruch 2, dadurch gekennzeichnet, daß die 2-Methoxy-4-amino-5-methylsulfamoylbenzoesäure aus 2-Methoxy-4-acetaminobenzoesäuremethylester durch aufeinanderfolgende Reaktionen mit Schwefelsäure, Methanol, $PCl_5$ und Methylamin und anschließende Hydrolyse des so erhaltenen Produktes hergestellt wird.

4. Benzamid nach Anspruch 1 als neues Arzneimittel.

5. Arzneimittel nach Anspruch 4 zur Verwendung in der grundlegenden Behandlung der Migräne.

6. Pharmazeutische Zusammensetzung, dadurch gekennzeichnet, daß sie als Wirkstoff das Benzamid nach Anspruch 1 zusammen mit einem pharmazeutisch verträglichen Träger enthält.

**Ansprüche** (für den Vertragsstaat AT)

1. Verfahren zur Herstellung von N-(1-Allyl-2-pyrrolidinylmethyl)-2-methoxy-4-amino-5-methylsulfamoylbenzamid sowie seinen quartären Ammoniumsalzen, N-Oxiden, optischen Isomeren und Additionssalzen mit pharmakologisch verträglichen Säuren, dadurch gekennzeichnet, daß man Chlorameisensaüreethylester mit 2-Methoxy-4-amino-5-methylsulfamoylbenzoesäure in Gegenwart von Triethylamin reagieren läßt und anschließend N-Allyl-2-aminoethylpyrrolidin mit dem so erhaltenen Anhydrid umsetzt.

2. Verfahren nach Anspruch 1, dadurch gekennzeichnet, daß man N-(1-Allyl-2-pyrrolidinylmethyl)-2-methoxy-4-amino-5-methylsulfamoylbenzamid mit einer organischen oder Mineralsäure in sein Additionssalz oder mit einem Alkylsalz in sein quartäres Ammoniumsalz umwandelt oder zu einem N-Oxid oxidiert.

3. Verfahren nach Anspruch 1, dadurch gekennzeichnet, daß man das Benzamid durch Behandlung mit einer optisch aktiven Säure in einer optisch aktiven Form erhält.

4. Verfahren nach Anspruch 1, dadurch gekennzeichnet, daß die 2-Methoxy-4-amino-5-methylsulfamoylbenzoesäure aus 2-Methoxy-4-acetaminobenzoesäuremethylester durch aufeinanderfolgende Reaktionen mit Schwefelsäure, Methanol, $PCl_5$ und Methylamin und anschließende Hydrolyse des so erhaltenen Produktes hergestellt wird.